# EUROPÄISCHE PATENTSCHRIFT

(11) **EP 1 648 551 B1**
(45) Veröffentlichungstag und Bekanntmachung des Hinweises auf die Patenterteilung: **17.02.2010**
(21) Anmeldenummer: 04741149.1
(22) Anmeldetag: 20.07.2004
(51) Int. Cl.: A61M 35/00, A61F 13/02, A61N 5/06, A61K 9/70

(54) **VORRICHTUNG ZUR VERBESSERUNG DER DURCHLÄSSIGKEIT DER MENSCHLICHEN HAUT**
DEVICE FOR IMPROVING THE PERMEABILITY OF THE HUMAN SKIN
DISPOSITIF POUR AMELIORER LA PERMEABILITE DE LA PEAU DE L'HOMME

(30) Priorität: 01.08.2003 DE 10335231
(43) Veröffentlichungstag der Anmeldung: 26.04.2006
(73) Patentinhaber: LTS LOHMANN Therapie-Systeme AG, 56626 Andernach (DE)
(72) Erfinder: HORSTMANN, Michael, 56564 Neuwied (DE)
(74) Vertreter: Schmidt, Werner
(86) Internationale Anmeldenummer: PCT/EP2004/008068
(87) Internationale Veröffentlichungsnummer: WO 2005/011797

(56) Entgegenhaltungen:
- EP-A- 0 294 122
- WO-A-98/58685
- DE-A- 10 128 629
- DE-A- 19 544 255
- DE-U- 29 612 198

## Beschreibung

Die Erfindung betrifft ein zumindest bereichsweise transparentes, wirkstoffhaltiges Pflaster gemäß dem Oberbegriff des Anspruchs 1. Dieses kann in Verbindung mit mindestens einer externen Lichtquelle zum Verbessern der Durchlässigkeit der menschlichen Haut für die transdermale Zufuhr von Wirkstoffen benutzt werden.

Transdermale therapeutische Systeme sind seit Jahren in der Therapie verschiedener topischer und systemischer Erkrankungen etabliert. Wirkstoffe, wie z.B. Nicotin, Estradiol Nitroglycerin, Fentanyl, können auf diese Weise wegen der deutlich verbesserten Pharmakokinetik und der Vermeidung des First-Pass-Effekts therapiegerechter appliziert werden als bei oraler Aufnahme. Die Auswahl der für die transdermale Zufuhr geeigneten Wirkstoffe ist jedoch limitiert. Bei einigen Wirkstoffen ist der Transport zwar möglich, die Formulierungen erfordern aber eine unpraktikabel große Fläche.

Eine mögliche Problemlösung stellen Permeationsverstärker dar. Diese Verstärker, z.B. Ethanol, Butanol und andere kurzkettige Alkohole, sind chemische Stoffe, die der Formulierung zugesetzt werden, um die Durchlässigkeit der menschlichen Haut vorübergehend zu erhöhen. Damit wird dann eine hinreichend hohe Flussrate des pharmazeutischen Wirkstoffes ermöglicht. Allerdings werden diese Verstärker vom Körper aufgenommen und belasten die metabolischen Vorgänge des Körpers.

In der DE 195 44 255 A, welche die Merkmale des Oberbegriffs von Anspruch 1 offenbart, wird ein Verbandsmaterial beschrieben, das ein mindestens bereichsweise transparent ausgebildetes Abdeckelement aufweist. Dieses Abdeckelement kann mindestens bereichsweise einen Klebebereich haben und ggf. mit einem Medikament versetzt oder getränkt sein. Durch das transparente Abdeckelement wird die Hautoberfläche z.B. mit farbigem Licht beleuchtet.

Ferner ist in der DE 101 28 629 A1 wird ein Wundpflaster beschrieben, das in seinem Zentrum eine künstliche Lichtquelle, einen Energiespeicher und eine Fresnellinse trägt. Das Hautpflaster wird z.B. auf eine Schnitt-, Schürf- oder Brandwunde geklebt. Die interne Lichtquelle beleuchtet über die Fresnellinse die menschliche Haut. Die bioverträgliche Bestrahlung der Wunde fördert den Heilungsprozess der Haut durch eine Beschleunigung der Wundschließung.

Der vorliegenden Erfindung liegt daher die Problemstellung zugrunde, ein Verfahren zur Verbesserung der Durchlässigkeit der menschlichen Haut zu entwickeln, das - ohne Nebenwirkungen für den Gesamtorganismus - eine reproduzierbare Durchlässigkeit für bestimmte Wirkstoffe ermöglicht.

Diese Problemstellung wird mit den Merkmalen des Haupt- und der Unteransprüche gelöst. Bei der Verwendung wird auf das erfindungsgemäße Pflaster zumindest bereichsweise normal auftreffendes, von einer externen Lichtquelle zumindest kurzfristig ausgesandtes Licht durch eine Vielzahl von einzelnen im Pflaster integrierten Sammellinsen auf die Hornschicht der Haut gebündelt, um so die Durchlässigkeit der Haut verbessernden Hornschichtveränderungen zu erzeugen.

Das Pflaster hat hierzu mindestens eine Deckschicht und mindestens eine wirkstoffhaltige selbstklebende Schicht. Die Deckschicht und die wirkstoffhaltige Schicht sind zumindest bereichsweise transparent, wobei die transparenten Bereiche innerhalb des Pflasters übereinander liegen und die Deckschicht eine Vielzahl flächig angeordneter optischer Sammellinsen umfasst.

Das transdermale therapeutische System besteht somit u.a. aus mindestens einer wirkstoffhaltigen hautzugewandten Matrixschicht und einer transparenten, geometrisch konturierten Deckschicht. Das System wird in Form eines Pflasters temporär auf die Körperhaut geklebt. Eine solche Anordnung erlaubt die Verwendung von Lichtquellen zur Verbesserung der transdermalen Resorption während der Tragedauer des Pflasters.

Weitere Einzelheiten der Erfindung ergeben sich aus den Unteransprüchen und der nachfolgenden Beschreibung schematisch dargestellter Ausführungsformen.
- Figur 1:: Pflaster und Hornhautschicht im Schnitt;
- Figur 2:: Teildraufsicht auf ein Linsenfeld ohne den Pflasterrand;
- Figur 3:: wie Figur 1, jedoch mit Linsen verschiedener Brennweiten;
- Figur 4:: wie Figur 1, jedoch mit mechanischer Falschlichtabschattung.

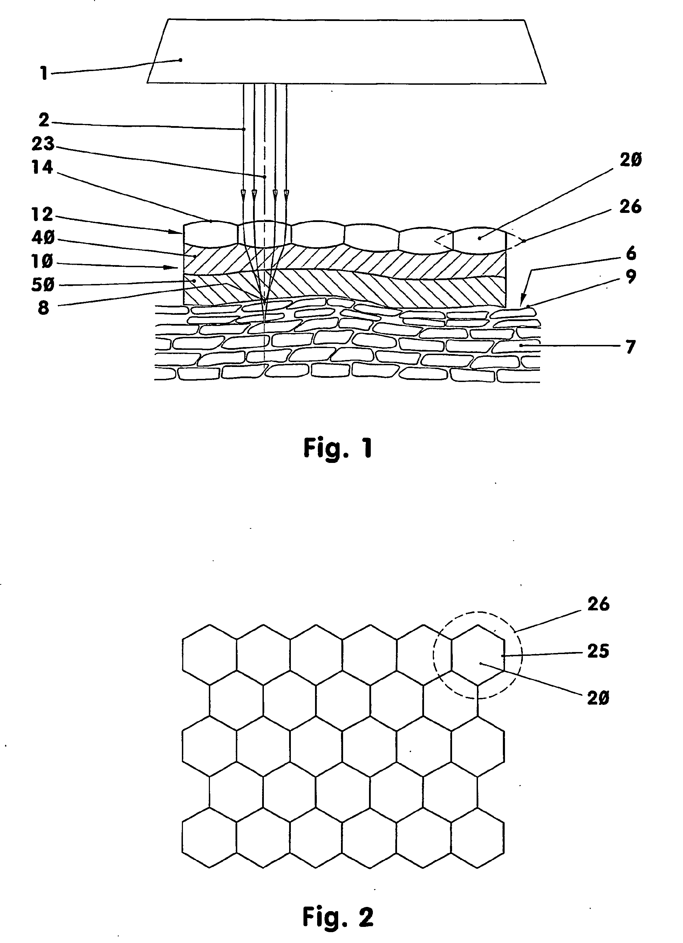

Die Figur 1 zeigt eine physikalische Methode, mit der die transdermale Wirkstoffzufuhr erheblich beschleunigt wird. Dazu wird z.B. ein selbstklebendes Pflaster (10) verwendet, das eine transparente Deck- bzw. Rückschicht (12, 13) und mindestes eine wirkstoffhaltige, ebenfalls transparente Klebe- bzw. Matrixschicht (50, 40) aufweist. Die Deckschicht (12) besteht aus einem Feld optischer Linsen (20-22). Das auf der Haut (6) aufgeklebte Pflaster (10) wird zumindest kurzfristig mit einer Lichtquelle (1) großer Beleuchtungsstärke belichtet. Das auf die Pflasterrückseite (14) zumindest nahezu senkrecht auftreffende Licht (2) wird von den einzelnen Sammellinsen (20-23) separat focusiert auf die Hornschicht (7) der Haut (6) projiziert. In den einzelnen Brennpunkten oder Brennlinien entstehen kleine Brennflecken (8), die die Hornschicht (7) für den Wirkstofftransport offen und dünn halten. Die Brennlinien entstehen aufgrund der Diakaustik der Sammellinsen.

Die Matrix- und/oder die Klebeschicht (40, 50) sind hierbei ein Wirkstoffdepot, das seinen Wirkstoff z.B. über Stunden oder Tage abgeben kann.

Das vor der Anwendung gelagerte Pflaster (10) ist zumindest durch eine auf der Klebeschicht (50) haftende Schutzfolie vor einer ungewollten Wirkstoffabgabe oder einem Wirkstoffverlust geschützt.

Die Deckschicht (12) ist hier z.B. eine transparente Folie, in der eine große Anzahl von kleinen Linsen (20) integriert ist. Jede einzelne Linse (20) hat beispielsweise eine doppeltkonvexe Form, deren Krümmungsmittelpunkte jeweils auf einer optischen Achse (23) liegen. Die einzelnen optischen Achsen (23) sind in der Regel normal zum jeweiligen Flächenelement der Plasterrückseite (14) orientiert. Der Abstand der optischen Achsen (23) zweier benachbarter Linsen (20) beträgt z.B. 50 bis 500 µm. In bestimmten Fällen kann der jeweilige Abstand bis auf einen Millimeter vergrößert werden. Die Brennweite der einzelnen Linsen (20) ist unter der Berücksichtigung der ggf. verschiedenen Brechungsindizes des Linsenwerkstoffs und der Matrixwerkstoffe so dimensioniert, dass die mittlere Brennweite ca. 10 bis 20 µm unter der äußeren Oberfläche (9) der Haut (6) in der Hornhautschicht (7) liegt. Bei einer 40 µm dicken Deckschicht (12) und einer 100 µm starken Matrix- und Klebeschicht (40, 50) beträgt z.B. die mittlere Brennweite somit 135 µm.

Radial sind die Linsen (20) nach Figur 2 beispielsweise durch die Mantelfläche (25) eines geraden, regelmäßigen und sechseckigen Primas begrenzt. Eine komplette Linse (20) hätte die in den Figuren 1 und 2 gezeigte kreisrunde Außenkontur (26). Alternativ können die Linsen (20) jeweils auch eine zylindrische Außenkontur haben. Die hierbei entstehenden Zwischenräume wären dann z.B. durch Planflächen ausgefüllt. Die mittlere Stärke der die Linsen (20) beinhaltenden flexiblen Folie (12) beträgt ca. 40 - 100 µm. Die Gesamtfläche des lichtdurchlässigen Teils des Pflasters (10) liegt je nach Anwendungsfall beispielsweise zwischen 2 und 50 cm².

Bei Anwendungsfällen, bei denen sich durch die Belichtung eine sichtbare partielle dunkle Tönung der Hornhaut ergibt, können in den äußeren Pflasterbereichen die Linsen z.B. teilweise eingetrübt oder ohne konvexe Krümmung ausgestattet sein, um den Übergangkontrast von ungetöntem zu getöntem Hornhautbereich beispielsweise aus kosmetischen Gründen zu verringern.

Dieses Prinzip lässt sich selbstverständlich auch umkehren. So kann durch eine bestimmte Anordnung von Brennpunkten erzeugende Linsen und optisch inaktiven Zwischenbereichen ein Bräunungsmuster in Form einer temporären Tätowierung auf der Hornhaut erzeugt werden.

Als Werkstoffe der Deckschicht sind denkbar: Polycarbonat, Polyethylen, Polymethylmethacrylat, Polyethylenterephthalat, und andere Polyester, Polypropylen, Acrylatpolymere, Polyamide, und anorganische Gläser oder dergleichen, sofern diese Materialen optisch brechende und transparente Eigenschaften haben.

Da in der Regel keine hohen Anforderungen an die optische Qualität der Linsenfelder gestellt wird, kann die Folie (12) z.B. im Spritzgussverfahren hergestellt werden. Bei Mikrolinsenfeldern mit überdurchschnittlich kleinen Linsen, kann die Folie (12) auch mikrolithografisch produziert werden.

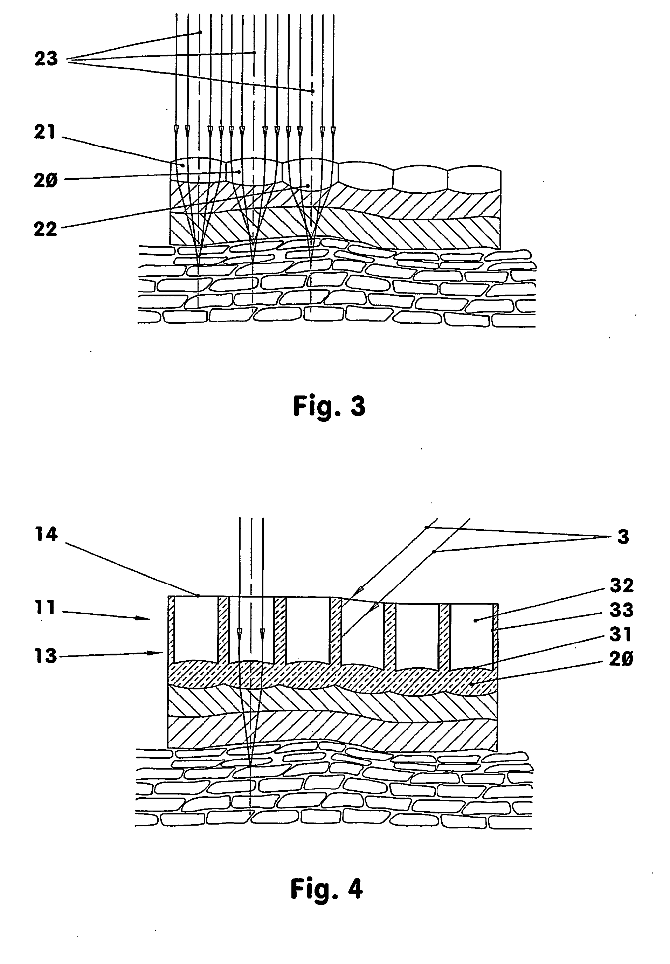

Nach Figur 3 können in einem Linsenfeld Linsen (20-22) verschiedener Brennweiten angeordnet sein. Im Ausführungsbeispiel werden drei verschiedene Linsen (20, 21, 22) verwendet, die innerhalb des Linsenfeldes z.B. regelmäßig verteilt angeordnet sind. Ihre Brennweiten variieren in einem Bereich von beispielsweise 10 bis 50 µm. Mit Hilfe eines derartigen Linsenfeldes kann eine dickere Hornhautschicht temporär durchlässiger gemacht werden.

In Figur 4 ist ein Pflaster dargestellt, dessen Deckschicht (13) z.B. der drei- bis vierfachen Materialstärke der Deckschicht (12) aus Figur 1 entspricht. Hier bilden die rückseitigen konvexen Flächen (31) der einzelnen Linsen (20) jeweils den Grund eines in der Deckschicht (13) eingelassenen Sackloches (32). Die Innenflächen (33) der Sacklöcher (32) - mit Ausnahme der Linsenoberfläche (31) - haben beispielsweise eine Beschichtung, die eine Totalreflektion entweder nicht oder nur in Form einer diffusen Reflektion zulässt. Ggf. besteht die Beschichtung aus einem mattschwarzen Farbauftrag. Hier kann dann schräg in die Sacklöcher (32) einfallendes Licht (3) unterhalb der Linse kaum eine Hautveränderung herbeiführen.

Anstelle des mit Sacklöchern (32) ausgestatteten Linsenfeldes kann auch ein aus Figur 1 bekanntes Linsenfeld verwendet werden, auf das ein flexibles Wabengitter aufgeklebt wird. Das Wabengitter, das ggf. aus einem anderen Werkstoff hergestellt wird als das Linsenfeld, besteht z.B. aus einer Vielzahl von Röhren mit sechseckigem Querschnitt. Die Mittellinien der Röhren sind weitgehend normal zur Hautoberfläche orientiert.

Eine andere Variante zur Eindämmung von Falschlicht besteht darin, an fast jedem einzelnen Rand der einzelnen Linsen (20-22) einen oder mehrere Zapfen anzuordnen, wobei die Zapfen im Wesentlichen normal zur Hautoberfläche (9) ausgerichtet sind. Die von der Pflasteraußenfläche (14) abstehenden Zapfen verschatten bei Falschlicht weitgehend die einzelnen Linsenoberflächen (31).

Eine weitere Alternative zur Dosierung der auf die Haut aufzubringenden Lichtmenge besteht in der Verwendung phototropischer Gläser. Derartige Linsenwerkstoffe dunkeln innerhalb von Sekunden bis Minuten die Linsen reversibel ab. Auch eine vollständige Abdeckung des Linsenfeldes mittels einer lichtundurchlässigen selbstklebenden Abdeckfolie ist vorstellbar.

Anstelle einer derartigen Mehrfachabblendung können auch Linsenwerkstoffe eingesetzt werden, die nach Minuten oder Stunden durch Alterung unter Lichteinwirkung sich dauerhaft eintrüben oder dunkel färben.

Mit einer definierten Belichtung der entsprechenden pflastertragenden Hornhautstelle wird eine Regelung des Wirkstofftransports durch die Haut reproduzierbar erzielt. Wichtige Einflussfaktoren sind hier beispielsweise eine konstante Strahlungsintensität und ein gleichbleibender Abstand zwischen der Lichtquelle und dem Pflaster. Da unter Umständen eine einmalige Licht- bzw. Strahlungsenergiezufuhr zu Anfang der Behandlung nicht ausreicht, kann es erforderlich sein, eine in gewissen Zeitabständen, z.B. Minuten oder Stunden, Lichtimpulse abgebende Blitzlampe zu konfigurieren, damit die durch die Brennlinsenwirkung entstandenen Strukturen der Haut offen gehalten bleiben.

### Bezugszeichenliste:

- 1: Lichtquelle
- 2: Licht, Lichtrichtung normal zur Pflasteroberfläche
- 3: Licht, Lichtrichtung schräg zur Pflasteroberfläche

- 6: Haut, menschlich
- 7: Hornschicht
- 8: Brennflecken, Hornschichtveränderungen
- 9: Oberfläche der Haut

- 10: Pflaster
- 11: Pflaster mit teilabgeschatteten Linsen
- 12, 13: Deckschicht, Rückschicht, Folie
- 13: Deckschicht mit Sacklöchern
- 14: Pflasteraußenseite, Pflasterrückseite

- 20-22: Linsen, Brenngläser
- 23: optische Achse
- 25: Mantelfläche
- 26: Außenkontur

- 31: Flächen, gekrümmt
- 32: Sacklöcher
- 33: Innenfläche, zylindrisch

- 40: Matrixschicht
- 50: Klebeschicht

## Patentansprüche

1. Pflaster für die transdermale Zufuhr von Wirkstoffen mit mindestens einer Deckschicht und mindestens einer wirkstoffhaltigen selbstklebenden Schicht,
wobei
die Deckschicht (12, 13) und die wirkstoffhaltige Schicht (40) zumindest bereichsweise transparent sind, wobei die transparenten Bereiche innerhalb des Pflasters. (10, 11) übereinander liegen, **dadurch gekennzeichnet,**
- **dass** die Deckschicht (12, 13) eine Vielzahl flächig angeordneter optischer Linsen (20-22) umfasst und
- **dass** die Brennpunkte der optischen Linsen (20 - 22) in der Hornschicht (7) der das Pflaster tragenden Haut (6) liegen.

2. Pflaster nach Anspruch 1,
**dadurch gekennzeichnet,**
**dass** die Schicht (40) eine Klebeschicht ist oder dass die Schicht (40) als ausschließlich wirkstoffhaltige Schicht mit einer zur Haut (6) hin orientierten, wirkstoffdurchlässigen Klebeschicht (50) ausgestattet ist.

3. Pflaster nach Anspruch 1,
**dadurch gekennzeichnet,**
**dass** mindestens ein Teil der optischen Linsen (20-22) unterschiedliche Brennweiten hat.

4. Pflaster nach Anspruch 1,
**dadurch gekennzeichnet,**
**dass** auf der der menschlichen Haut (6) abgewandten Seite des Pflasters (11) auf dem Feld aus optischen Linsen (20-22) abschattende Erhebungen angeordnet sind.

## Claims

1. Plaster for transdermal delivery of active substances, with at least one top layer and at least one self-adhesive layer that contains active substance,
- the top layer (12, 13) and the layer (40) containing active substance being transparent in at least some areas, the transparent areas lying over one another inside the plaster (10, 11), **characterized in that**
- the top layer (12, 13) comprises a multiplicity of optical lenses (20-22) organized in a planar arrangement and
- the focal points of the optical lenses (20-22) lie in the stratum corneum (7) of the skin (6) bearing the plaster.

2. Plaster according to Claim 1, **characterized in that** the layer (40) is an adhesive layer, or **in that** the layer (40), as an exclusively active-substance-containing layer, is provided with an adhesive layer (50) which is permeable to active substance and oriented toward the skin (6).

3. Plaster according to Claim 1, **characterized in that** at least some of the optical lenses (20-22) have different focal lengths.

4. Plaster according to Claim 1, **characterized in that**, on that side of the plaster (11) directed away from the human skin (6), shading elevations are arranged on the array of optical lenses (20-22).

## Revendications

1. Sparadrap pour l'administration transcutanée d'agents actifs comprenant au moins une couche de protection et au moins une couche autocollante contenant l'agent actif, la couche de protection (12, 13) et la couche autocollante contenant l'agent actif (40) étant au moins localement transparentes, les zones transparentes étant superposées à l'intérieur du sparadrap (10, 11), **caractérisé en ce que**
- la couche de protection (12, 13) comprend une pluralité de lentilles optiques (20-22) disposées à plat et
- que les points focaux des lentilles optiques (20-22) se trouvent dans la couche de corne (7) de la peau (6) qui supporte le sparadrap.

2. Sparadrap selon la revendication 1, **caractérisé en ce que** la couche (40) est une couche adhésive ou que la couche (40) est réalisée exclusivement sous la forme d'une couche contenant l'agent actif avec une couche adhésive (50) perméable à l'agent actif orientée vers la peau (6).

3. Sparadrap selon la revendication 1, **caractérisé en ce qu'**au moins une partie des lentilles optiques (20-22) présentent des distances focales différentes.

4. Sparadrap selon la revendication 1, **caractérisé en ce que** des bossages faisant de l'ombre aux lentilles optiques (20-22) sont disposés sur le côté du sparadrap (11) à l'opposé de la peau humaine (6).
